# EUROPEAN PATENT APPLICATION

(11) **EP 4 431 142 A1**
(43) Date of publication of application: **18.09.2024**
(21) Application number: 22892643.2
(22) Date of filing: 31.10.2022
(51) Int. Cl.: A61M 31/00, A61M 5/142

(54) **ADMINISTRATION DEVICE**

(30) Priority: 10.11.2021 JP 2021183294
(71) Applicant: TERUMO Kabushiki Kaisha, Tokyo 151-0072 (JP)
(72) Inventor: YOSHIKAWA, Hiroki, Ashigarakami-gun, Kanagawa 259-0151 (JP); HATA, Suguru, Ashigarakami-gun, Kanagawa 259-0151 (JP); YAMAMOTO, Satoshi, Ashigarakami-gun, Kanagawa 259-0151 (JP)
(74) Representative: Casalonga
(86) International application number: PCT/JP2022/040617
(87) International publication number: WO 2023/085144

(57) **Abstract**

A device that is indwelled in an in-vivo lumen and releases a substance X to be administered that is accommodated, the device including: the cylindrical main body portion 10 having the inner cavity 11 in which the substance X to be administered can be accommodated and configured to be able to retain a shape of the inner cavity 11 against an external force; and an ejection portion 40 that is slidable in a liquid-tight manner in the inner cavity 11,in which at least a part of the main body portion 10 is composed of the deformable member that is deformable along a shape of the in-vivo lumen.

## Description

### BACKGROUND

### Technical Field

The present invention relates to an administration device that administers a substance to be administered such as a drug solution to an administration target in a state where the device is indwelled in a living body.

### Related Art

JP 2021-66747 A discloses an osmotic delivery device that is a continuous administration device that continuously releases, in a sustained manner for a long period of time, a drug that is a substance to be administered in a state where the device is embedded in a living body for a long period of time.

The device of JP 2021-66747 A is roughly constituted with a hollow container (main body portion), a piston (ejection portion) slidably held in the container, an osmotic engine (pressing portion) serving as a drive source for pressing the piston, a semipermeable membrane (liquid permeation portion) that is disposed on a proximal-end-side of the container and allows a liquid component in a body fluid (extracellular fluid) to permeate the osmotic engine, and a delivery orifice (release portion) that is disposed on a distal end side of the container and releases a drug pressed by the piston. The container is partitioned into two spaces by the piston, and includes a drug containing space (second space) positioned on the distal end side from the piston, of the container, and an osmotic preparation containing space (first space) positioned on a proximal-end-side from the piston, of the container, which is an opposite side of the second space, and is constituted to be able to expand an osmotic preparation by the liquid component in the body fluid that has permeated the semipermeable membrane, slide the piston toward the distal end side as an internal pressure of the first space increases due to expansion of the osmotic preparation, and release the drug contained in the second space into the living body, using the principle of osmotic pressure.

### [Citation List]

### [Patent Literature]

Patent Literature 1: JP 2021-66747 A.

### SUMMARY

The device of JP 2021-66747 A is intended to be indwelled in a living body and continuously administer a drug stored in a limited space over a long period of time. Therefore, in the device of JP 2021-66747 A, the entire main body portion is made of a rigid member (such as titanium) so as to cause the piston to stably slide while securing a sliding space for the piston when indwelled in the living body.

However, in a case where the device of JP 2021-66747 A is indwelled in a blood vessel and the substance to be administered is to be delivered to the delivery site transvascularly, since the main body portion constituting the device housing is composed of a rigid material, it is difficult to pass the substance to be administered depending on the shape of the blood vessel, and there is a risk that the blood vessel wall is damaged when the main body portion is forced to pass.

Alternatively, by reducing the length and the tube diameter of the main body portion, it is possible to pass through the curved site of the blood vessel. However, when the size of the main body portion is reduced, the volume for accommodating the substance to be administered is also reduced, causing a new problem that the sustained release period is shortened.

At least one embodiment of the present invention has been made in view of the above circumstances, and specifically, an object of the present invention is to provide an administration device capable of easily conveying to a target indwelling position without damaging an in-vivo lumen while securing a sufficient accommodation volume of a substance to be administered when indwelled in the in-vivo lumen.

An administration device according to the present embodiment is indwelled in an in-vivo lumen and releases a substance to be administered that is accommodated, the administration device includes: a cylindrical main body portion having an inner cavity in which the substance to be administered can be accommodated and configured to be able to retain a shape of the inner cavity against an external force; and an ejection portion that is slidable in a liquid-tight manner in the inner cavity, in which at least a part of the main body portion is composed of a deformable member that is deformable along a shape of the in-vivo lumen.

According to at least one embodiment of the present invention, since the administration device has the main body portion configured to be deformable following the shape of the in-vivo lumen, it is possible to convey or indwell the administration device to a target indwelling position without damaging the in-vivo lumen while securing a sufficient accommodation volume of the substance to be administered without reducing the device size. Therefore, the administration device can deliver the substance to be administered to the delivery site transvascularly under desired administration conditions while being indwelled in the in-vivo lumen.

### BRIEF DESCRIPTION OF DRAWINGS

FIG.1 is a schematic cross-sectional view of the administration device according to the present embodiment;
FIG. 2A is a schematic view illustrating a connection mode between a flexible site and a rigid site of the administration device according to the present embodiment;
FIG. 2B is a schematic view illustrating another connection mode between a flexible site and a rigid site of the administration device according to the present embodiment;
FIG. 2C is a schematic view illustrating another connection mode between a flexible site and a rigid site of the administration device according to the present embodiment;
FIG. 3A is a conceptual diagram illustrating an operation when the administration device according to the present embodiment is indwelled in a blood vessel which is an in-vivo lumen;
FIG. 3B is a conceptual diagram illustrating an operation when the administration device according to the present embodiment is indwelled in a blood vessel which is an in-vivo lumen;
FIG. 3C is a conceptual diagram illustrating an operation when the administration device according to the present embodiment is indwelled in a blood vessel which is an in-vivo lumen;
FIG. 4A is a schematic configuration view illustrating a first modification of the administration device according to the present embodiment;
FIG. 4B is a schematic configuration view illustrating a second modification of the administration device according to the present embodiment; and
FIG. 4C is a schematic configuration view illustrating a third modification of the administration device according to the present embodiment.

### DETAILED DESCRIPTION

Hereinafter, a mode for carrying out the present invention will be described in detail with reference to the drawings. Embodiments herein are illustrated to embody the technical idea of the present invention and do not limit the present invention. Furthermore, other modes, examples, operation techniques, and the like, that could be conceived of by those skilled in the art without departing from the gist of the present invention are all included in the scope and gist of the present invention and included in the invention set forth in the claims and the scope of equivalents thereof.

Moreover, for convenience of illustration and ease of understanding, the drawings attached to the present specification may be schematically represented by changing a scale, an aspect ratio, a shape, and the like, from actual ones as appropriate, but are merely examples, and do not limit the interpretation of the present invention.

Note that, in the following description, ordinal numerals such as "first" and "second" will be given, but are used for convenience and do not define any order unless otherwise specified.

The administration device 100 according to the present embodiment is a device for administering (sustainedly releasing) the substance X to be administered such as a drug to a predetermined delivery site in a living body. The administration device 100 is placed in an in-vivo lumen (such as a blood vessel Bv) in order to deliver the substance X to be administered to a delivery site in the living body, and can release the substance X to be administered under predetermined administration conditions (conditions related to administration such as administration period, administration timing, and dose). In the present embodiment, the administration device 100 is configured as a continuous administration device that performs continuous administration (sustained release) over a long period of time (at least weeks to months, even years).

Note that the administration device 100 is not limited to the configuration described below, and is not particularly limited as long as it is indwelled in the blood vessel Bv and can release X under a predetermined administration condition.

The administration device 100 of the present embodiment employs an osmotic engine that expands by the liquid component of the body fluid in the living body, as the configuration of the pressing portion 30 that presses the ejection portion 40. However, in the administration device 100, the pressing portion 30 is not limited to the osmotic engine, and may be a device employing, for example, a mechanical configuration using a motor as a drive source.

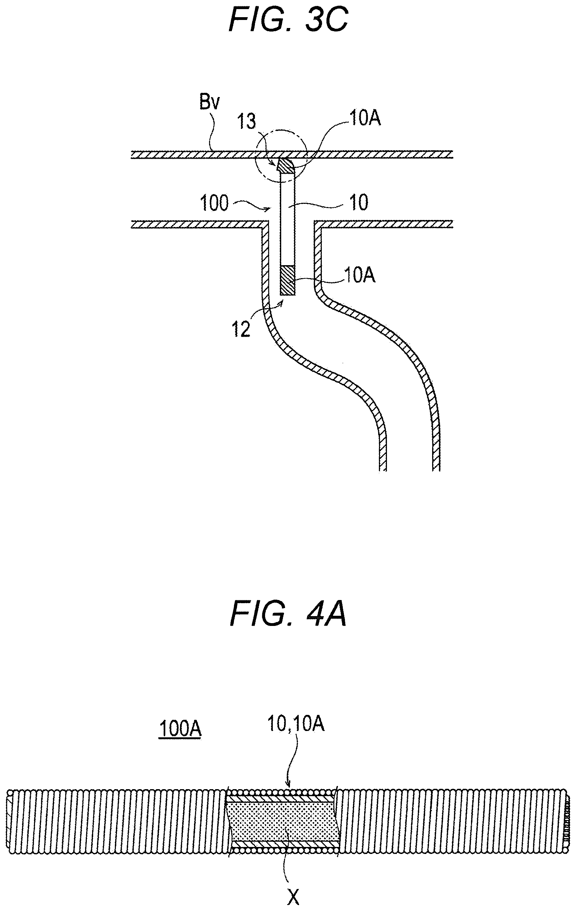

The substance X to be administered from the administration device 100 is a fluid composition that can express a predetermined effect by sustained release to the living body that is the administration target and can be intermittently released from the device. The substance X to be administered is, for example, a medical agent (liquid preparation) intended for treatment of a predetermined disease. The medical agent includes a drug. The drug can be any physiologically or pharmacologically active substance, in particular one known to be delivered to a human or animal body. The drug includes, but is not limited to, a drug acting on a peripheral nerve, an adrenergic receptor, a cholinergic receptor, a skeletal muscle, a cardiovascular system, a smooth muscle, a vascular system, a synoptic site, a nerve exchanger junction site, an endocrine and hormonal system, an immune system, a reproductive system, a skeletal system, a local hormonal system, a digestive and excretory system, a histamine system, or a central nervous system. In addition, the drug includes, but is not limited to, a drug to be used for treatment of infectious diseases, chronic pain, diabetes, autoimmune diseases, endocrine diseases, metabolic abnormalities, and rheumatic diseases. In addition, the drug includes, but is not limited to, peptides, protein, polypeptides (for example, enzyme, hormone, cytokine), nucleic acid, oligonucleotides, viruses, viral vectors, plasmids, nucleoprotein, polysaccharide, glycoprotein, lipoprotein, cells, steroid, analgesic, a local anesthetic, an antibiotic formulation, an anti-inflammatory corticosteroid, eye drops, other small molecules for pharmaceutical use, or synthetic analogs of these species, and mixtures thereof, and the like. As an example, the administration device 100 is indwelled in the blood vessel Bv of a patient and used (see FIG. 3C).

### <Configuration>

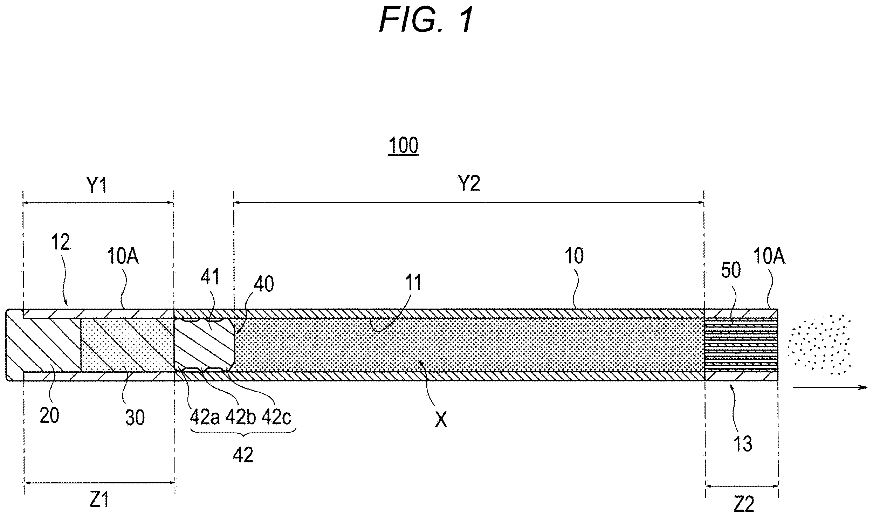

As illustrated in FIG. 1, the administration device 100 generally includes a main body portion 10, a liquid permeation portion 20, a pressing portion 30, an ejection portion 40, and a release portion 50.

The administration device 100 of the present embodiment employs an osmotic engine that expands by the liquid component of the body fluid in the living body, as the configuration of the pressing portion 30 that presses the ejection portion 40. However, in the administration device 100, the pressing portion 30 is not limited to the osmotic engine, and may be a device employing, for example, a mechanical configuration using a motor as a drive source. In addition, the administration device 100 is not particularly limited as long as it can be indwelled in the in-vivo lumen and includes the main body portion 10 having at least the inner cavity 11 and the ejection portion 40 capable of liquid-tightly sliding in the inner cavity 11, and at least a part of the main body portion 10 is formed of the deformable member described later.

The main body portion 10 is constituted with a hollow cylindrical member including the inner cavity 11 and constituting a housing of the administration device 100. The main body portion 10 has a proximal end 12 which is a site located on an upstream side (left side in FIG. 1) into which a liquid component in a body fluid (extracellular fluid) flows and a distal end 13 which is a site located on a downstream side (right side in FIG. 1) from which the substance X to be administered is released to the living body. The liquid permeation portion 20, the pressing portion 30, the ejection portion 40, and the release portion 50 are arranged in order from the proximal end 12 toward the distal end 13 of the main body portion 10.

The main body portion 10 has a first space Y1 and a second space Y2 partitioned by the ejection portion 40. The first space Y1 is a space defined by the liquid permeation portion 20 and the ejection portion 40 (a space between the attachment end of the liquid permeation portion 20 and the proximal end of the ejection portion 40 in the main body portion 10), and is a space in which the pressing portion 30 is accommodated. The second space Y2 is a space defined by the ejection portion 40 and the release portion 50 (a space between the proximal end of the ejection portion 40 and the proximal end of the release portion 50), and is a space in which the ejection portion 40 and the substance X to be administered are accommodated. The second space Y2 constitutes a sliding region of the ejection portion 40.

Spaces of the first space Y1 and the second space Y2 are enlarged or reduced as the ejection portion 40 slides before and after the administration device 100 is driven. In other words, in the first space Y1, if the administration device 100 starts to be driven, the ejection portion 40 slides toward the downstream side by the pressing portion 30, and an interval gradually increases. In addition, in the second space Y2, if the administration device 100 starts to be driven, the ejection portion 40 slides toward the downstream side by the pressing portion 30, and an interval gradually decreases.

At least a part of the main body portion 10 is composed of the flexible deformable member that is deformable along a shape of the in-vivo lumen (blood vessel Bv). The site of the main body portion 10 composed of the deformable member functions as a deformable portion 10A that is deformable along the shape of the blood vessel Bv. In the present embodiment, in the main body portion 10, the liquid permeation portion 20, a proximal-end-side accommodation region Z1 (corresponding to the region of the first space Y1) in which the pressing portion 30 is accommodated, and a distal-end-side accommodation region Z2 in which the release portion 50 is accommodated are configured by the deformable portion 10A formed of the deformable member. That is, in the main body portion 10 of the present embodiment, sites other than the sliding region of the ejection portion 40 are composed of the deformable portion 10A. By composing at least a part of the main body portion 10 of the deformable member, the main body portion is deformable along a shape of the blood vessel through which the main body portion passes when conveyed to the indwelling position or a shape of the blood vessel at the indwelling position.

The deformable member only needs at least such flexibility that deformation following the blood vessel shape is possible, and may be composed of a flexible material having flexibility, or may have a structure (flexible structure) capable of being deformed following the blood vessel shape.

As the flexible material constituting the deformable member, a resin material used for a catheter or the like known in the medical field can be used, and as an example, a polymer material such as polyolefin (for example, polyethylene, polypropylene, polybutene, an ethylenepropylene copolymer, an ethylene-vinyl acetate copolymer, an ionomer, a mixture of two or more thereof, or the like), polyvinyl chloride, polyamide, polyester elastomer, polyamide elastomer, polyurethane, polyurethane elastomer, polyimide, or a fluororesin such as polytetrafluoroethylene (PTFE) can be used. In addition, a fiber, a coil, a spring, or the like made of metal, resin, or the like may be combined as a reinforcing member.

In addition, the deformable member can be configured by a structure that can be flexibly deformed following the shape of the in-vivo lumen such as a bellows structure. The deformable member may have a flexible structure composed of the flexible material, or may be composed of a material having a certain degree of hardness. That is, the material and structure of the site functioning as the deformable portion 10A of the main body portion 10 are not particularly limited as long as the configuration allows deformation along the blood vessel shape at the time of conveyance or indwelling.

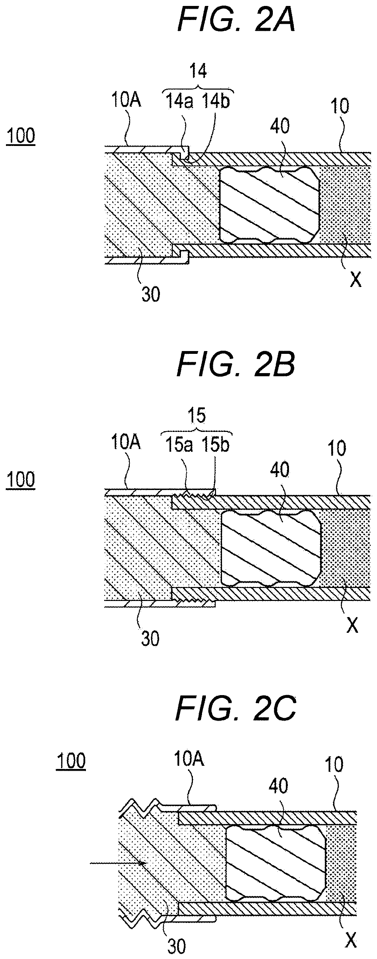

FIGS. 2A to 2C illustrate joining modes between a site functioning as the deformable portion 10A in the main body portion 10 (site corresponding to the proximal-end-side accommodation region Z1 or the distal-end-side accommodation region Z2: hereinafter, simply referred to as "flexible site") and a site having higher rigidity than the deformable member in the main body portion 10 (site corresponding to the second space Y2: hereinafter, simply referred to as "rigid site").

As illustrated in FIG. 2A, the joining mode between the flexible site and the rigid site can be in a configuration in which an engagement claw 14a is provided at a distal end of the flexible site, an engagement hole 14b is provided in an outer peripheral surface on a proximal end side of the rigid site, and an engagement portion 14 for connecting and fixing by engaging the engagement claw 14a and the engagement hole 14b is provided. In addition, as another joining mode, as illustrated in FIG. 2B, a configuration in which a thread groove 15a is formed on the inner peripheral surface of the distal end of the flexible site, a thread ridge 15b is formed on the outer peripheral surface of the rigid site, and a screwing portion 15 for connecting and fixing by screwing together the thread groove 15a and the thread ridge 15b is provided can be adopted. As another joining mode, as shown in FIG. 2C, a configuration in which the distal end of the flexible site is press-fitted into the distal end of the rigid site to be connected and fixed may be adopted, and further, connection by an adhesive or the like may also be adopted. In addition, the flexible site and the rigid site may be connected by providing a connection band on an outer layer of the connection portion. The connecting band may cover not only the connection portion but also the rigid site. The connecting band may cover up to the flexible site if it is a sufficiently flexible material. Note that the joining mode between the flexible site and the rigid site is not limited to the above-described modes, and may be any mode as long as the connection between the flexible site and the rigid site can be maintained when indwelled in the in-vivo lumen and the like.

By composing at least a part of the main body portion 10 of the deformable member, the main body portion is deformable following the shape of the blood vessel Bv when conveyed to the indwelling position or indwelled, so that the main body portion can be conveyed to a target indwelling position while securing a sufficient accommodation volume of the substance to be administered without reducing the device size. In addition, composing the proximal end 12 and the distal end 13 of the main body portion 10 of the deformable members provides an effect whereby, even if the edge end comes into contact with the inner wall surface of the blood vessel Bv when the main body portion is conveyed in the blood vessel Bv, the main body portion is flexibly deformed to reduce the risk of damage to the blood vessel wall.

As illustrated in FIG. 1, both the proximal end 12 and the distal end 13 of the main body portion 10 are composed of the deformable member, but it is only required that at least one of the proximal end 12 and the distal end 13 is composed of the deformable member. As a result, the administration device 100 can be conveyed along the blood vessel shape or indwelled in the blood vessel Bv without damaging the blood vessel wall since the site (the proximal end 12 or the distal end 13) composed of the deformable member can be deformed along the blood vessel shape.

In the main body portion 10, in order to maintain the release accuracy of the substance X to be administered by the sliding of the ejection portion 40, it is necessary to prevent at least the inner cavity 11 in the sliding region of the ejection portion 40 from being crushed by buckling or the like and the shape of the inner cavity 11 (cross-sectional shape intersecting the axial direction) from being deformed to inhibit the sliding of the ejection portion 40. As a constituent material of the main body portion 10, a resin material (acrylonitrile polymers, halogenated polymers, polyimide, polysulfone, polycarbonate, polyethylene, polypropylene, polyvinyl chloride-acrylic acid copolymer, polycarbonate-acrylonitrile-butadiene-styrene, polystyrene, and the like) or a metal material (stainless steel, titanium, nickel, aluminum, vanadium, platinum, tantalum, gold, and alloys thereof, and gold plated alloy iron, platinum plated alloy iron, cobalt chromium alloy, titanium nitride-coated stainless steel, and the like) which is non-invasive or minimally invasive to a living body and known in the medical field and the like can be applied.

Furthermore, as will be described in detail later, the main body portion 10 may be composed of the flexible deformable member other than the above-described material. In this case, the main body portion 10 may have a structure capable of retaining at least the shape of the inner cavity 11 to be the sliding region of the ejection portion 40 against an external force, or may be provided with a reinforcing member (see FIGS. 4A to 4C).

Referring again to FIG. 1, the liquid permeation portion 20 is disposed on the proximal-end-side of the main body portion 10, isolates the living body from inside of the administration device 100, and permeates only a liquid component contained in a body fluid in the living body. The liquid permeation portion 20 is constituted with a member having a solid-liquid separation function capable of permeating only a liquid component in a body fluid.

As an example, a semipermeable membrane including a plasticized cellulose-based material, reinforced polymethyl methacrylates (PMMA) such as hydroxyl ethyl methacrylate (HEMA), and elastomer materials such as polyurethanes and polyamides, polyether-polyamide copolymers, and thermoplastic copolyesters can be applied as the liquid permeation portion 20.

The pressing portion 30 is located on the downstream side with respect to the liquid permeation portion 20 in the main body portion 10 and presses the ejection portion 40 toward the downstream side. In the present embodiment, the pressing portion 30 is an osmotic engine that expands by being permeated by the liquid component that has permeated the liquid permeation portion 20 using the principle of osmotic pressure. The pressing portion 30 is gradually expanded by the liquid component that has permeated the liquid permeation portion 20 and slides the ejection portion 40 toward the downstream side by pressing action caused by increase in an internal pressure in the first space Y1 due to the expansion.

A constituent material, a size, and the like, of the pressing portion 30 can be appropriately determined so as to achieve a pressing speed (that is, an expansion speed of the pressing portion 30) of the ejection portion 40 according to use conditions (a sustained release period, a release amount per unit time, and the like, of the substance X to be administered). The administration device 100 can adjust the sustained release period and the sustained release amount by the configuration of the osmotic engine composing the pressing portion 30, a thickness of the semipermeable membrane composing the liquid permeation portion 20, and the like.

The ejection portion 40 is a plug (piston) including a columnar base portion 41 and an annular protruding portion 42 protruding from a radially outer periphery of the base portion 41. The ejection portion 40 is pressed by the pressing portion 30 and moves to the downstream side to push out the substance X to be administered stored in the second space Y2 toward the release portion 50. The ejection portion 40 is located on the downstream side with respect to the pressing portion 30 in the main body portion 10, has an outer peripheral surface in liquid-tight contact with the inner peripheral surface of the inner cavity 11 of the main body portion 10 and is slidably disposed in the inner cavity 11.

In the present embodiment, the protruding portion 42 includes a first protruding portion 42a provided on an outer surface on the proximal-end-side of the base portion 41, a second protruding portion 42b provided substantially in the middle of the base portion 41, and a third protruding portion 42c provided on the outer surface on the distal end side of the base portion 41. An apex portion protruding outermost of the protruding portion 42 abuts on the inner peripheral surface of the inner cavity 11 of the main body portion 10. The first protruding portion 42a, the second protruding portion 42b, and the third protruding portion 42c seal between the inner peripheral surface of the inner cavity 11 and the first protruding portion 42a, the second protruding portion 42b, and the third protruding portion 42c so that the substance X to be administered does not leak to the proximal-end-side with respect to the ejection portion 40.

Although the protruding portion 42 includes the first protruding portion 42a, the second protruding portion 42b, and the third protruding portion 42c, the number and arrangement positions thereof are not particularly limited. In addition, it is also possible to employ a configuration where the ejection portion 40 does not include the protruding portion 42.

The material composing the ejection portion 40 is a flexible material that maintains adherence (liquid tightness) with respect to the inner peripheral surface of the inner cavity 11 of the main body portion 10. The flexible material is preferably an elastic material, and examples of the elastic material include various rubber materials (in particular, those subjected to vulcanization treatment) such as natural rubber, isoprene rubber, butyl rubber, chloroprene rubber, nitrile-butadiene rubber, styrene-butadiene rubber, and silicone rubber, styrene-based elastomers, hydrogenated styrene-based elastomers, and styrene-based elastomers mixed with polyolefins such as polyethylene, polypropylene, polybutene, and α-olefin copolymers, oils such as liquid paraffin and process oil, and powder inorganic substances such as talc, cast, and mica. Furthermore, a polyvinyl chloride-based elastomer, an olefin-based elastomer, a polyester-based elastomer, a polyamide-based elastomer, a polyurethane-based elastomer, or a mixture thereof can be used as the constituent material. As the constituent material, in particular, a diene-based rubber, a styrene-based elastomer, or the like can be applied from the viewpoint of having elastic characteristics and enabling γ-ray sterilization, electron beam sterilization, and high-pressure steam sterilization. The ejection portion 40 only requires to be slidable in the inner cavity 11 in a liquid-tight manner. Thus, the base portion 41 and the protruding portion 42 of the ejection portion 40 may be constituted with a flexible material or an elastic material, or for example, only the protruding portion 42 abutting on the inner cavity 11 may be constituted with an elastic material, and the base portion 41 may be constituted with a hard material having no elasticity.

The release portion 50 is disposed on the distal end side of the main body portion 10, and releases the substance X to be administered pushed out by the ejection portion 40 into the living body. The release portion 50 has a plurality of through holes penetrating a plate-like member having a predetermined thickness along an axial direction in order to efficiently administer the substance X to be administered into the living body. The release portion 50 has a plurality of through holes, so that the substance X to be administered can be diffused into the living body without being solidified at one place. Note that a shape and the number of the through holes formed in the release portion 50 can be appropriately determined in consideration of properties of the substance X to be administered, ease of diffusion of the substance X to be administered at the place where the administration device 100 is indwelled, and the like.

In addition, the administration device 100 can be attached with a delivery catheter (cannula) (not illustrated) that delivers the substance X to be administered released from the release portion 50 to the delivery site. The proximal end side of the delivery catheter is connected to the distal end 13, and the distal end side is fixed in a state of being introduced to the delivery site, for example. As a result, the administration device 100 can deliver the substance X to be administered to the delivery site transvascularly in a state of being indwelled in the blood vessel Bv.

### <Operation>

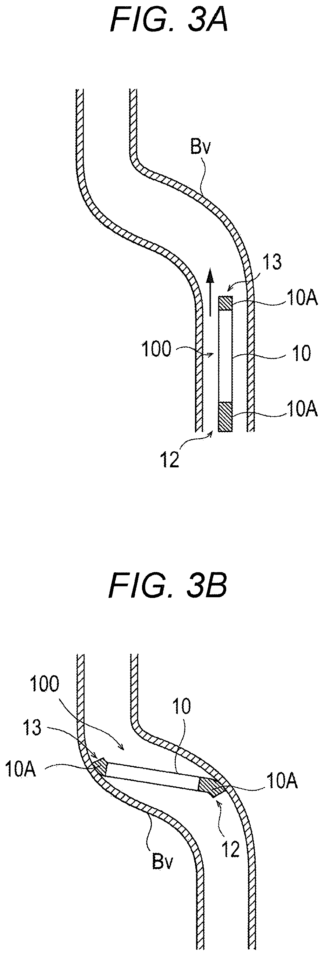

Next, an operation example of the administration device 100 according to the present embodiment will be described with reference to FIGS. 3A to 3C.

The administration device 100 is introduced percutaneously into the blood vessel Bv after being filled with the substance X to be administered for the administration target. As illustrated in FIG. 3A, the administration device 100 may pass through a curved site of the blood vessel Bv when being conveyed from the puncture position (introduction position) of the skin to the indwelling position.

At this time, the operator continues advancing the administration device 100 along the blood vessel Bv. As illustrated in FIG. 3B, the administration device 100 deforms along the shape of the blood vessel Bv by the deformable portion 10A positioned at the proximal end 12 or the distal end 13 being pressed against the blood vessel wall in the process of passing through the curved site of the blood vessel Bv. In addition, when passing through the curved site of the blood vessel Bv, the administration device 100 maintains the shape of the inner cavity 11 of the portion corresponding to the sliding region of the main body portion 10.

Thereafter, the administration device 100 is delivered to a target indwelling position (a portion enclosed by a dashed-dotted line in the drawing). As illustrated in FIG. 3C, when the indwelling position is a curved site, the administration device 100 can be indwelled in a state where the distal end 13 is deformed along the shape of the curved site of the blood vessel Bv, for example. In this manner, in the administration device 100, at least a part of the main body portion 10 can be deformed along the shape of the curved site of the blood vessel Bv when conveyed from the introduction position to the indwelling position or indwelled in the blood vessel Bv. Therefore, the administration device 100 can be conveyed or indwelled without damaging the blood vessel Bv while securing a sufficient capacity of the substance X to be administered without reducing the device size.

If a predetermined period elapses after the administration device 100 is indwelled at the indwelling position, the liquid component in the body fluid permeates into the liquid permeation portion 20. The pressing portion 30 starts to expand by the liquid component that has permeated from the liquid permeation portion 20. The ejection portion 40 starts sliding toward the downstream side of the second space Y2 as a result of the internal pressure of the inner cavity 11 increasing as the pressing portion 30 expands. After the initial sliding, the administration device 100 shifts to the normal sliding and gradually slides toward the downstream side. In this event, the ejection portion 40 slides to the downstream side at a substantially constant sliding speed so that a prescribed amount of the substance X to be administered is continuously administered for a long period of time. In the administration device 100, the sliding of the ejection portion 40 is stopped in sync with the end timing of the administration period of the substance X to be administered, and the distal end of the ejection portion 40 comes into contact with the surface on the proximal end side of the release portion 50. When the administration period of the substance X to be administered ends, the administration device 100 is extracted from the living body.

### [Modifications]

Next, modifications of the administration device 100 will be described with reference to FIGS. 4A to 4C as appropriate. In each of the modifications described below, constituent elements having the same functions as those in the above-described embodiment may be denoted by the same reference numerals and detailed description thereof may be omitted, and configurations, members, usage methods, and the like, that are not particularly mentioned may be similar to those in the above-described embodiment. Further, the configurations of the present embodiment and the configurations of each of the modifications can be appropriately merged without departing from and contradicting the gist of the present invention.

### <First modification>

As illustrated in FIG. 4A, the administration device 100A of the first modification includes the deformable member in which the main body portion 10 has flexibility and the shape of the inner cavity 11 can be retained against an external force. In the administration device 100A of the first modification, the entire main body portion 10 functions as a deformable portion 10A. For example, a coil tube can be applied as the deformable member in the main body portion 10 of the first modification. The coil tube has a structure in which a long wire material made of metal (stainless steel, Ni, Ti, an alloy thereof, or the like) or resin is spirally wound around a peripheral surface (outer peripheral surface or inner peripheral surface) of a tube main body made of a flexible resin material. Therefore, the main body portion 10 is deformed along the blood vessel shape, but the shape (cross-sectional shape) of the inner cavity 11 is retained by the long wire material.

In the administration device 100A of the first modification, the main body portion 10 is configured by the deformable member (coil tube) capable of retaining the shape of the inner cavity 11 while having flexibility. Since the administration device 100A can be deformed while maintaining the shape of the inner cavity 11 along the blood vessel shape at the time of conveyance inside the blood vessel Bv, it is possible to be conveyed inside the blood vessel Bv to the target indwelling position without inhibiting the sliding of the ejection portion 40. In addition, in the administration device 100A, since the entire main body portion 10 functions as the deformable portion 10A, the main body portion 10 can be freely deformed, and it is not necessary to reduce the size and diameter size of the main body portion 10 according to the blood vessel shape serving as the conveyance path. Therefore, the administration device 100A can be manufactured in a size capable of accommodating the required amount of the substance X to be administered.

### <Second modification>

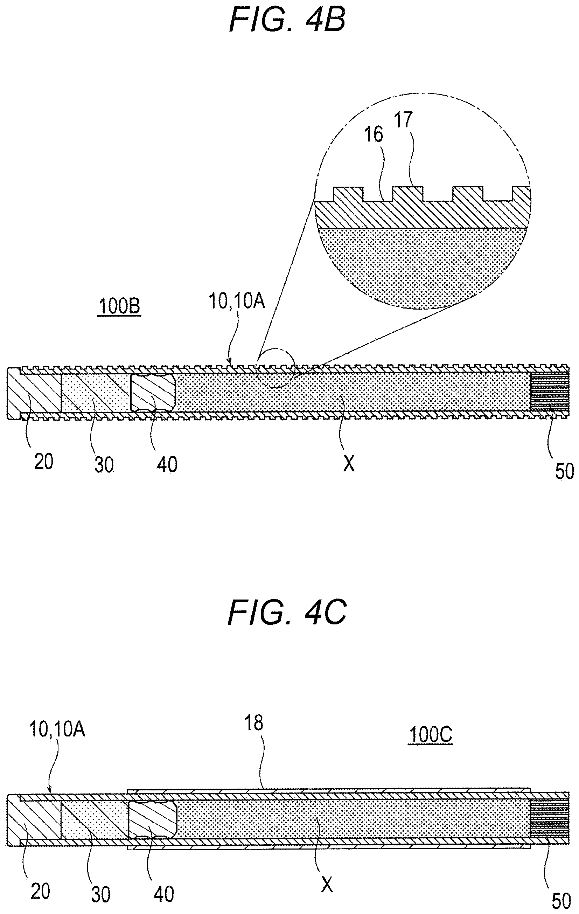

As illustrated in FIG. 4B, the administration device 100B of the second modification is configured by the deformable member having a flexible structure in which a thin portion 16 in which the thickness of the main body portion 10 is thinned along the axial direction and a thick portion 17 having a thickness to such an extent that the shape (cross-sectional shape) of the inner cavity 11 can be retained against an external force are alternately arranged. In the administration device 100B of the second modification, the entire main body portion 10 functions as a deformable portion 10A. For example, a flexible tube can be applied as the deformable member in the main body portion 10 of the second modification.

In the main body portion 10 of the second modification, by alternately arranging the thin portion 16 and the thick portion 17 along the axial direction, the thin portion 16 is deformed and can be deformed along the blood vessel shape. In addition, since the main body portion 10 of the second modification has the thick portion 17, the shape of the inner cavity 11 can be held against an external force. The constituent material of the main body portion 10 of the second modification is not particularly limited as long as at least the thin portion 16 is formed of a material deformable along the blood vessel shape.

In the administration device 100B of the second modification, the main body portion 10 is configured by the deformable member having a flexible structure and capable of retaining the shape of the inner cavity 11 while having flexibility. Since the administration device 100B can be deformed while maintaining the shape of the inner cavity 11 along the blood vessel shape at the time of conveyance inside the blood vessel Bv, it is possible to be conveyed inside the blood vessel Bv to the target indwelling position without inhibiting the sliding of the ejection portion 40. In addition, in the administration device 100B, since the entire main body portion 10 functions as the deformable portion 10A, the main body portion 10 can be freely deformed, and it is not necessary to reduce the size and diameter size of the main body portion 10 according to the blood vessel shape serving as the conveyance path. Therefore, the administration device 100B can be manufactured in a size capable of accommodating the required amount of the substance X to be administered.

### <Third modification>

As shown in FIG. 4C, in the administration device 100C of the third modification, the main body portion 10 is made of the flexible material that can be deformed along the blood vessel shape, and is composed of the deformable member having a reinforcing portion 18 on the outer peripheral surface along the axial direction so that the shape (cross-sectional shape) of the inner cavity 11 is held against an external force in a site corresponding to the sliding region of the ejection portion 40. In the administration device 100C of the third modification, the entire main body portion 10 functions as a deformable portion 10A. In the main body portion 10 of the third modification, since the reinforcing portion 18 is disposed in a site corresponding to the sliding region of the ejection portion 40, the shape of the inner cavity 11 can be retained against an external force even if the main body portion is deformed.

In the deformable member of the third modification, the reinforcing portion 18 may be the flexible material or a hard material having rigidity higher than that of the flexible material. When the flexible material is used for the reinforcing portion 18, the strength may be increased by, for example, providing a thickness so that the shape of the inner cavity 11 can be retained against an external force.

In the administration device 100C of the third modification, the main body portion 10 is composed of the deformable member in which the entire tube is composed of the flexible material and the reinforcing portion 18 is provided in a site corresponding to the sliding region. Since the administration device 100C can be deformed while maintaining the shape of the inner cavity 11 along the blood vessel shape at the time of conveyance inside the blood vessel Bv, it is possible to be conveyed inside the blood vessel Bv to the target indwelling position without inhibiting the sliding of the ejection portion 40. In addition, in the administration device 100C, since the entire main body portion 10 functions as the deformable portion 10A, the main body portion 10 can be freely deformed, and it is not necessary to reduce the size and diameter size of the main body portion 10 according to the blood vessel shape serving as the conveyance path. Therefore, the administration device 100C can be manufactured in a size capable of accommodating the required amount of the substance X to be administered.

Further, as another modification, the main body portion 10 may be made of a material having rigidity, and for example, a joint portion (or a neighboring region including the joint portion) between a site corresponding to the proximal-end-side accommodation region Z1 or the distal-end-side accommodation region Z2 illustrated in FIG. 1 and another site of the main body portion 10 adjacent to the proximal-end-side accommodation region Z1 or the distal-end-side accommodation region Z2 may be made of the deformable member. According to such a mode, since the deformable member at the joint portion functions as the deformable portion 10A, the main body portion 10 can be deformed along the blood vessel shape from the deformable portion 10A as a starting point, and the same effects as those of the above-described embodiments and each of the modifications can be obtained.

### [Operational Effects]

As described above, the administration device 100 according to the present embodiment is indwelled in an in-vivo lumen and releases a substance X to be administered that is accommodated, the administration device including: the cylindrical main body portion 10 having the inner cavity 11 in which the substance X to be administered can be accommodated and configured to be able to retain a shape of the inner cavity 11 against an external force; and an ejection portion 40 that is slidable in a liquid-tight manner in the inner cavity 11,in which at least a part of the main body portion 10 is composed of the deformable member that is deformable along a shape of the in-vivo lumen.

With such a configuration, since the administration device 100 has the main body portion 10 configured to be deformable following the shape of the in-vivo lumen such as the blood vessel Bv, it is possible to convey or indwell the administration device to a target indwelling position without damaging the blood vessel Bv while securing a sufficient accommodation volume of the substance to be administered without reducing the device size. In addition, since the administration device 100 has a configuration in which the shape of the inner cavity 11 is retained, the flow rate accuracy of the substance X to be administered by the ejection portion 40 is retained. Therefore, the administration device 100 can deliver the substance X to be administered to the delivery site transvascularly under desired administration conditions while being indwelled in the blood vessel Bv.

In addition, in the administration device 100 according to the present embodiment, the deformable member may be made of the flexible material, or may be made of a member having a structure deformable along the shape of the in-vivo lumen.

Since at least a part of the main body portion 10 is composed of the flexible material or the deformable member having the structure deformable along the shape of the blood vessel Bv (flexible structure), the administration device 100 can be conveyed to the indwelling position while being deformed along the shape of the blood vessel Bv without damaging the blood vessel wall, or can be appropriately indwelled at the indwelling position.

In addition, in the administration device 100 according to the present embodiment, at least one of the proximal end 12 and the distal end 13 of the main body portion 10 may be composed of the deformable member.

Since at least one of the proximal end 12 and the distal end 13 of the main body portion 10 is composed of the deformable member, the administration device 100 can be smoothly conveyed along the shape of the blood vessel Bv without damaging the blood vessel wall. In addition, even when the indwelling position is the curved site of the blood vessel Bv, the administration device 100 can be appropriately indwelled without damaging the blood vessel wall by deforming the proximal end 12 and the distal end 13 along the curved shape of the blood vessel Bv.

In the administration device 100 according to the present embodiment, in the main body portion 10, the proximal end 12 which is a site on an upstream side of the sliding region of the ejection portion 40 in the inner cavity 11 and accommodates the liquid permeation portion 20 permeating a liquid and the pressing portion 30 pressing the ejection portion 40 by an action of the liquid that has permeated the liquid permeation portion 20, and the distal end 13 which is a site on a downstream side of the sliding region and accommodates the release portion 50 discharging to the outside the substance X to be administered pressed by sliding of the ejection portion 40, are composed of the deformable member, and the site corresponding to the sliding region may be composed of a material harder than the deformable member capable of retaining the shape of the inner cavity 11 against an external force.

Since the proximal end 12 accommodating the liquid permeation portion 20 and the pressing portion 30 in the main body portion 10 and the distal end 13 accommodating the release portion 50 are composed of the deformable member, the administration device 100 can be smoothly conveyed along the shape of the blood vessel Bv without damaging the blood vessel wall. In addition, even when the indwelling position is the curved site of the blood vessel Bv, the administration device 100 can be appropriately indwelled without damaging the blood vessel wall by deforming the proximal end 12 and the distal end 13 along the curved shape of the blood vessel Bv. Furthermore, in the administration device 100, since the site corresponding to the sliding region of the ejection portion 40 is made of a hard material, the shape of the inner cavity 11 is retained, and the flow rate accuracy of the substance X to be administered by the ejection portion 40 can be maintained.

The present application is based on Japanese Patent Application No. 2021-183294 filed on November 10, 2021, the disclosure content of which is incorporated herein by reference in its entirety.

### [Reference Signs List]

10 Main body portion (10A Deformable portion)
11 Inner cavity
12 Proximal end of the main body
13 Distal end of the main body
20 Liquid permeation portion
30 Pressing portion
40 Ejection portion
50 Release portion
100 Administration device
Y1 First space
Y2 Second space
Z1 Proximal-end-side accommodation region
Z2 Distal-end-side accommodation region
Bv Blood vessel (In-vivo lumen)
X Substance

## Claims

1. An administration device that is indwelled in an in-vivo lumen and releases a substance to be administered that is accommodated, the administration device comprising:
a cylindrical main body portion having an inner cavity in which the substance to be administered can be accommodated and configured to be able to retain a shape of the inner cavity against an external force; and
an ejection portion that is slidable in a liquid-tight manner in the inner cavity,
wherein at least a part of the main body portion is composed of a deformable member that is deformable along a shape of the in-vivo lumen.

2. The administration device according to claim 1, wherein the deformable member is made of a flexible material.

3. The administration device according to claim 1 or 2, wherein the deformable member is composed of a member having a structure deformable along the shape of the in-vivo lumen.

4. The administration device according to any one of claims 1 to 3, wherein at least one of a proximal end and a distal end of the main body portion is composed of the deformable member.

5. The administration device according to claim 4, wherein:
in the main body portion,
the proximal end which is a site on an upstream side of a sliding region of the ejection portion in the inner cavity and accommodates a liquid permeation portion permeating a liquid and a pressing portion pressing the ejection portion by an action of the liquid that has permeated the liquid permeation portion, and
the distal end which is a site on a downstream side of the sliding region and accommodates a release portion discharging to the outside the substance to be administered pressed by sliding of the ejection portion, are composed of the deformable member; and
the site corresponding to the sliding region is composed of a material harder than the deformable member capable of retaining the shape of the inner cavity against an external force.
